# EUROPÄISCHE PATENTSCHRIFT

(11) **EP 2 792 649 B1**
(45) Veröffentlichungstag und Bekanntmachung des Hinweises auf die Patenterteilung: **27.11.2019**
(21) Anmeldenummer: 13163828.0
(22) Anmeldetag: 15.04.2013
(51) Int. Cl.: C03C 3/097, A61L 27/10, A61K 6/027, C03C 3/112, C03C 10/00

(54) **Lithiumsilikat-Glaskeramik und -Glas mit Gehalt an Rubidiumoxid**
Lithium silicate glass ceramic and glass with rubidium oxide content
Vitrocéramique et verre au lithium-silice ayant une teneur en oxyde de rubidium

(43) Veröffentlichungstag der Anmeldung: 22.10.2014
(73) Patentinhaber: Ivoclar Vivadent AG, 9494 Schaan (LI)
(72) Erfinder: Ritzberger, Christian, 9472 Grabs (CH); Rampf, Markus, 8853 Lachen (CH); Dittmer, Marc, 6800 Feldkirch (AT); Höland, Wolfram, 9494 Schaan (LI); Schweiger, Marcel, 7000 Chur (CH); Rheinberger, Volker, 9490 Vaduz (LI)
(74) Vertreter: Uexküll & Stolberg

(56) Entgegenhaltungen:
- EP-A1- 1 514 850
- EP-A1- 1 688 397
- EP-A1- 2 377 830
- WO-A1-2012/175450
- WO-A2-2013/053863
- US-A- 3 732 087

## Beschreibung

Die Erfindung betrifft Lithiumsilikat-Glaskeramik und Lithiumsilikatglas, die Rubidiumoxid enthalten und sich durch einen in einem breiten Bereich von insbesondere 9,0 bis 14,0·10⁻⁶ K⁻¹ einstellbaren linearen thermischen Ausdehnungskoeffizienten auszeichnen und deshalb vor allem im Dentalbereich zur Verblendung von Oxidkeramikrestaurationen und von Metallrestaurationen eignen.

In der Zahnheilkunde werden Dentalrestaurationen üblicherweise mit keramischen Schichten verblendet, um das Aussehen der Restauration an das der natürlichen Zähne anzugleichen. Solche verblendeten Restaurationen werden auch als Verblendkeramiken bezeichnet. Um Spannungen zwischen dem zu verblendenden Restaurationsmaterial und der Keramikschicht zu vermeiden, ist es erforderlich, dass die Wärmeausdehnungskoeffizienten der keramischen Werkstoffe an die des Restaurationsmaterials angepasst sind.

Zur Beschichtung oder Verblendung von Oxidkeramiken, wie etwa Zirkonoxidkeramiken, sind in der Vergangenheit bereits Glaskeramiken eingesetzt worden. Dazu zählen auf Feldspat basierende Keramiken oder Fluoroapatit-Glaskeramiken.

Weiter sind Lithiumdisilikat-Glaskeramiken bekannt, die aufgrund ihrer hohen Transluzenz und sehr guten mechanischen Eigenschaften besonders im Dentalbereich und dabei vornehmlich zur Herstellung von Dentalkronen und kleinen Brücken Anwendung finden. Die bekannten Lithiumsilikat-Glaskeramiken enthalten üblicherweise als Hauptkomponenten SiO₂, Li₂O, Al₂O₃, Na₂O oder K₂O und Keimbildner wie P₂O₅.

WO 2013/053863 A2 beschreibt Rb₂O enthaltende Lithiumsilikat-Glaskeramiken und -Gläser.

EP 0 885 855 A2 und EP 0 885 856 A2 beschreiben apatithaltige Glaskeramiken mit ausgezeichneten optischen und chemischen Eigenschaften und einer Festigkeit im Bereich von 110 MPa, die sich zur Verblendung von ZrO₂-Gerüsten eignen.

WO 2004/021921 A1 beschreibt ein Glas zur Verblendung von ZrO₂, welches jedoch nur geringe Festigkeit aufweist.

EP 1 253 116 A1 beschreibt eine Mischung aus einem Lithiumsilikatglas mit Leucitkristallen zur Verblendung von Metallgerüsten. Auch dieses Glas weist nur eine ungenügende Festigkeit auf.

WO 2012/082156 A1 beschreibt ein Lithiumsilikat-Produkt zur Verblendung von Metallgerüsten mit einem Ausdehnungskoeffizienten WAK_{100-400°C} von 12 bis 13,5·10⁻⁶ K⁻¹ und Festigkeiten von bis zu 300 MPa. EP 2 377 831 A1 beschreibt eine Lithiumsilikat-Glaskeramik mit Gehalten an ZrO₂. Der Ausdehnungskoeffizient der Glaskeramik ist nicht für die Verblendung von Metallgerüsten geeignet.

Damit eine Dentalglaskeramik zum Verblenden der gesamten Bandbreite der üblicherweise verwendeten Restaurationsmaterialien, wie Dentalmetallen und -legierungen bis hin zu Oxidkeramiken eingesetzt werden kann, ist es erforderlich, dass ihr Ausdehnungskoeffizient in einem breiten Bereich einstellbar ist. Zudem müssen die Glaskeramiken hohen Anforderungen in Bezug auf ihre optischen und mechanischen Eigenschaften genügen und insbesondere eine sehr hohe Festigkeit aufweisen.

Bekannte Glaskeramiken und Gläser erfüllen die Forderung nach in einem weiten Bereich einstellbaren thermischen Ausdehnungskoeffizienten und ausreichender Festigkeit häufig nicht. Weiter sind bei den bekannten Glaskeramiken regelmäßig das Erdalkalimetalloxid BaO sowie die Alkalimetalloxide K₂O und/oder Na₂O als essentielle Komponenten vorhanden, die dort zur Erzeugung der Glaskeramiken und insbesondere der Bildung der meist angestrebten Lithiumdisilikat-Hauptkristallphase offenbar erforderlich sind.

Es besteht daher ein Bedarf an Lithiumsilikat-Glaskeramiken, bei denen der lineare thermische Ausdehnungskoeffizient WAK_{100-400°C} über einen breiten Bereich und insbesondere im Bereich von 9,0 bis 14,0·10⁻⁶ K⁻¹ und vorzugsweise im Bereich von 9,6 bis 12,8·10⁻⁶ K⁻¹ einstellbar ist. Weiter sollen sie auch ohne die bisher als erforderlich angesehenen Alkalimetalloxide K₂O oder Na₂O sowie insbesondere ohne das Erdalkalimetalloxid BaO herstellbar sein und sich aufgrund vor allem ihrer optischen und mechanischen Eigenschaften insbesondere zur Verblendung von dentalen Restaurationen einschließlich Oxidkeramikrestaurationen und Metallrestaurationen eignen.

Diese Aufgabe wird durch die Verwendung einer Lithiumsilikat-Glaskeramik oder eines Lithiumsilikatglases nach einem der Ansprüche 1 bis 14 gelöst. Gegenstand der Erfindung sind ebenfalls das Verfahren nach den Ansprüchen 15 bis 20, der Verbundwerkstoff nach Anspruch 21, die Lithiumsilikat-Glaskeramik nach Anspruch 22 und das Lithiumsilikatglas nach Anspruch 23.

Die erfindungsgemäße Verwendung zeichnet sich dadurch aus, dass eine Lithiumsilikat-Glaskeramik oder ein Lithiumsilikatglas, die die folgenden Komponenten enthalten

| Komponente | Gew.-% |
|---|---|
| SiO₂ | 56,0 bis 72,0 |
| Li₂O | 13,0 bis 18,0 |
| Rb₂O | 3,0 bis 9,0 |
| Al₂O₃ | 2,0 bis 5,0 |
| P₂O₅ | 2,0 bis 6,0 |
| ZrO₂ | 0 bis 4,5, |

zur Beschichtung eines Substrats ausgewählt aus Oxidkeramiken, Metallen und Legierungen verwendet werden.

Es hat sich überraschend gezeigt, dass die erfindungsgemäße Lithiumsilikat-Glaskeramik einen linearen thermischen Ausdehnungskoeffizienten WAK_{100-400°C} aufweist, der leicht in einem breiten Bereich von insbesondere 9,0 bis 14,0·10⁻⁶ K⁻¹ und vorzugsweise 9,6 bis 12,8·10⁻⁶ K⁻¹ einstellbar ist, und zudem hervorragende optische und mechanische Eigenschaften wie hohe Festigkeit und Bruchzähigkeit aufweist. Diese Glaskeramik ist daher sowohl zur Beschichtung von Oxidkeramiken als auch von Metallen und Legierungen geeignet. Besonders überraschend ist, dass dabei die Bildung einer Glaskeramik mit Lithiummeta- und/oder -disilikat als Hauptkristallphase auch bei Abwesenheit verschiedener bei konventionellen Glaskeramiken als erforderlich angesehener Komponenten wie insbesondere K₂O, Na₂O und BaO gelingt. Die Bildung der erfindungsgemäßen Glaskeramik kann auch durch die Verwendung des erfindungsgemäßen Lithiumsilikatglases erzielt werden, welches einen Vorläufer für die Lithiumsilikat-Glaskeramik darstellt und vor, während oder nach dem Aufbringen auf das Substrat in diese umgewandelt werden kann.

Es ist bevorzugt, dass die erfindungsgemäß verwendete Lithiumsilikat-Glaskeramik und das erfindungsgemäß verwendete Lithiumsilikatglas mindestens eine und bevorzugt alle der folgenden Komponenten in den angegebenen Mengen enthalten

| Komponente | Gew.-% |
|---|---|
| SiO₂ | 56,9 bis 72,0 |
| Li₂O | 14,2 bis 18,0 |
| Rb₂O | 3,7 bis 7,7 |
| Al₂O₃ | 2,5 bis 4,5 |
| P₂O₅ | 3,1 bis 5,0 |
| ZrO₂ | 0 bis 4,0 |
| Übergangsmetalloxid | 0 bis 7,5, insbesondere 0 bis 7,0, |

wobei das Übergangsmetalloxid ausgewählt ist aus der Gruppe bestehend aus Oxiden von Yttrium, Oxiden von Übergangsmetallen mit Ordnungszahl 41 bis 79 und Mischungen dieser Oxide.

Vorzugsweise enthalten die Lithiumsilikat-Glaskeramik und das Lithiumsilikatglas 58,0 bis 72,0, insbesondere 60,0 bis 71,0 und bevorzugt 63,0 bis 70,0 Gew.-% SiO₂.

Auch ist es bevorzugt, dass die Lithiumsilikat-Glaskeramik und das Lithiumsilikatglas 14,4 bis 17,5, insbesondere 14,5 bis 17,0 und besonders bevorzugt 14,8 bis 16,0 Gew.-% Li₂O enthalten.

In einer bevorzugten Ausführungsform beträgt das Molverhältnis von SiO₂ zu Li₂O 2,0 bis 3,0, insbesondere 2,2 bis 2,6, bevorzugt 2,3 bis 2,5 und besonders bevorzugt etwa 2,4. In einer anderen bevorzugten Ausführungsform beträgt das Molverhältnis von SiO₂ zu Li₂O weniger als 2,0, insbesondere 1,5 bis 1,9, bevorzugt 1,6 bis 1,8 und besonders bevorzugt etwa 1,7.

Es ist bevorzugt, dass die Lithiumsilikat-Glaskeramik und das Lithiumsilikatglas 3,7 bis 7,7 Gew.-%, insbesondere 5,1 bis 7,7 Gew.-% und bevorzugt 6,1 bis 7,4 Gew.-% Rb₂O enthalten.

Auch ist es bevorzugt, dass die Lithiumsilikat-Glaskeramik und das Lithiumsilikatglas 2,5 bis 4,0 Gew.-%, insbesondere 3,0 bis 3,5 Gew.-% und bevorzugt 3,2 bis 3,4 Gew.-% Al₂O₃ enthalten.

Vorzugsweise beträgt das Molverhältnis von Rb₂O zu Al₂O₃ mindestens 0,1, insbesondere 0,2 bis 2,0, bevorzugt 0,25 bis 1,25 und besonders bevorzugt 0,5 bis 1,0.

Vorzugsweise enthalten das Glas und die Glaskeramik als Keimbildungsmittel 3,2 bis 4,5 Gew.-% und insbesondere 3,4 bis 4,0 Gew.-% P₂O₅.

Die erfindungsgemäß verwendete Lithiumsilikat-Glaskeramik und das erfindungsgemäß verwendete Lithiumsilikatglas können darüber hinaus noch Zusatzkomponenten enthalten, die insbesondere ausgewählt sind aus weiteren Oxiden einwertiger Elemente, Oxiden zweiwertiger Elemente, weiteren Oxiden dreiwertiger Elemente, weiteren Oxiden vierwertiger Elemente, weiteren Oxiden fünfwertiger Elemente, Oxiden sechswertiger Elemente, Schmelzbeschleunigern, Färbemitteln und Fluoreszenzmitteln. In einer bevorzugten Ausführungsform enthalten die Lithiumsilikat-Glaskeramik und das Lithiumsilikatglas Zusatzkomponenten in einer Menge von 0 bis 20,0 Gew.-%, insbesondere 0,1 bis 10,0 Gew.-%, bevorzugt 0,5 bis 7,5 Gew.-% und am meisten bevorzugt 1,0 bis 5,0 Gew.-%.

Der Begriff "weitere Oxide einwertiger Elemente" bezeichnet Oxide einwertiger Elemente und insbesondere Alkalimetalloxide mit Ausnahme von Li₂O und Rb₂O. Beispiele für geeignete weitere Oxide einwertiger Elemente sind Na₂O, K₂O, Cs₂O und Mischungen davon und insbesondere Na₂O, K₂O und Mischungen davon.

In einer Ausführungsform enthalten die Lithiumsilikat-Glaskeramik und das Lithiumsilikatglas 0,1 bis 2,0 Gew.-%, insbesondere 0,2 bis 1,5 Gew.-%, bevorzugt 0,3 bis 1,4 Gew.-% und besonders bevorzugt 0,5 bis 1,0 Gew.-% Na₂O. In einer weiteren Ausführungsform enthalten die Lithiumsilikat-Glaskeramik und das Lithiumsilikatglas 0,1 bis 2,0 Gew.-%, insbesondere 0,2 bis 1,6 Gew.-%, bevorzugt 0,4 bis 1,5 Gew.-% und besonders bevorzugt 0,5 bis 1,0 Gew.-% K₂O. In einer besonders bevorzugten Ausführungsform enthalten die Lithiumsilikat-Glaskeramik und das Lithiumsilikatglas weniger als 4,0 Gew.-%, insbesondere weniger als 3,5 Gew.-%, bevorzugt weniger als 3,0 Gew.-%, besonders bevorzugt weniger als 2,5 Gew.-% und am meisten bevorzugt weniger als 2,0 Gew.-% Na₂O und/oder K₂O.

Vorzugsweise enthalten die Lithiumsilikat-Glaskeramik und das Lithiumsilikatglas weniger als 2,5 Gew.-%, insbesondere weniger als 1,5 Gew.-%, bevorzugt weniger als 1,0 Gew.-%, besonders bevorzugt weniger als 0,5 Gew.-% Cs₂O. Am meisten bevorzugt sind sie im Wesentlichen frei von Cs₂O.

Als Oxide zweiwertiger Elemente kommen insbesondere die Erdalkalimetalloxide, vorzugsweise MgO, CaO, SrO, BaO und Mischungen davon, und bevorzugt CaO, SrO und Mischungen davon in Frage.

Vorzugsweise enthalten die Lithiumsilikat-Glaskeramik und das Lithiumsilikatglas weniger als 3,8 Gew.-%, insbesondere weniger als 2,5 Gew.-%, bevorzugt weniger als 1,5 Gew.-%, besonders bevorzugt weniger als 0,5 Gew.-% BaO. Am meisten bevorzugt sind sie im Wesentlichen frei von BaO.

Der Begriff "weitere Oxide dreiwertiger Elemente" bezeichnet Oxide dreiwertiger Elemente mit Ausnahme von Al₂O₃. Geeignete Oxide dreiwertiger Elemente sind insbesondere Y₂O₃, La₂O₃, Bi₂O₃ und Mischungen davon, und bevorzugt Y₂O₃ und La₂O₃.

Der Begriff "weitere Oxide vierwertiger Elemente" bezeichnet Oxide vierwertiger Elemente mit Ausnahme von SiO₂. Beispiele für geeignete weitere Oxide vierwertiger Elemente sind TiO₂, GeO₂ und ZrO₂.

Der Begriff "weitere Oxide fünfwertiger Elemente" bezeichnet Oxide fünfwertiger Elemente mit Ausnahme von P₂O₅. Beispiele für geeignete weitere Oxide fünfwertiger Elemente sind Ta₂O₅ und Nb₂O₅.

Beispiele für geeignete Oxide sechswertiger Elemente sind WO₃ und MoO₃.

Bevorzugt sind ein Glas und eine Glaskeramik, die mindestens ein weiteres Oxid einwertiger Elemente, ein Oxid zweiwertiger Elemente, mindestens ein weiteres Oxid dreiwertiger Elemente, mindestens ein weiteres Oxid vierwertiger Elemente, mindestens ein weiteres Oxid fünfwertiger Elemente und/oder mindestens ein Oxid sechswertiger Elemente enthalten.

Beispiele für Schmelzbeschleuniger sind Fluoride.

Beispiel für Färbemittel und Fluoreszenzmittel sind Oxide von d- und f-Elementen, wie z.B. die Oxide von Ti, V, Sc, Mn, Fe, Co, Ta, W, Ce, Pr, Nd, Tb, Er, Dy, Gd, Eu und Yb. Als Färbemittel können auch Metallkolloide, z.B. von Ag, Au und Pd, verwendet werden, die zusätzlich auch als Keimbildner fungieren können. Diese Metallkolloide können z.B. durch Reduktion von entsprechenden Oxiden, Chloriden oder Nitraten während der Schmelz- und Kristallisationsprozesse gebildet werden. Die Metallkolloide sind vorzugsweise in einer Menge von 0,005 bis 0,5 Gew.-% in der Glaskeramik enthalten.

Die erfindungsgemäß verwendete Glaskeramik weist als Hauptkristallphase vorzugsweise Lithiummetasilikat und/oder Lithiumdisilikat auf. Der Begriff "Hauptkristallphase" bezeichnet dabei die Kristallphase, die gegenüber den anderen Kristallphasen den höchsten Volumenanteil hat. Wenn zwei Kristallphasen annähernd gleichen Volumenanteil haben, können diese Kristallphasen beide als Hauptkristallphasen vorliegen. In anderen Ausführungsformen kann Lithiummetasilikat als Hauptkristallphase und Lithiumdisilikat als Nebenphase oder Lithiumdisilikat als Hauptkristallphase und Lithiummetasilikat als Nebenphase vorliegen.

Es hat sich überraschenderweise gezeigt, dass die erfindungsgemäße Lithiumsilikat-Glaskeramik sehr gute mechanische und optische Eigenschaften aufweist, auch wenn bei konventionellen Glaskeramiken als wesentlich angesehene Komponenten fehlen. Die Kombination ihrer Eigenschaften erlaubt es sogar, sie als Dentalmaterial und insbesondere zur Beschichtung von Dentalrestaurationen einzusetzen.

Die erfindungsgemäße Lithiumsilikat-Glaskeramik hat vorzugsweise eine Bruchzähigkeit, gemessen als K_{IC} Wert, von mindestens etwa 2,0 MPa·m^{0,5} und insbesondere mindestens etwa 2,3 MPa·m^{0,5}. Dieser Wert wurde mit dem Vicker's-Verfahren bestimmt und mittels Niihara-Gleichung berechnet. Weiter hat sie eine hohe biaxiale Bruchfestigkeit von bevorzugt 180 bis 700 MPa. Überdies zeigt sie eine hohe chemische Beständigkeit, die durch Masseverlust nach Lagerung in Essigsäure ermittelt wurde. Die chemische Beständigkeit beträgt insbesondere weniger als 100 µg/cm². Die biaxiale Bruchfestigkeit und die chemische Beständigkeit wurden gemäß ISO 6872 (2008) bestimmt.

In einer bevorzugten Ausführungsform enthält die Glaskeramik Lithiummetasilikat als Hauptkristallphase. Insbesondere enthält die Glaskeramik mehr als 5 Vol.-%, bevorzugt mehr als 10 Vol.-% und besonders bevorzugt mehr als 15 Vol.-% an Lithiummetasilikat-Kristallen, bezogen auf die gesamte Glaskeramik. Diese Lithiummetasilikat-Glaskeramik zeichnet sich durch sehr gute mechanische Eigenschaften aus. Vorzugsweise weist sie eine Biegefestigkeit im Bereich von etwa 180 bis 300 MPa und/oder eine Bruchzähigkeit, gemessen als K_{IC} Wert, von mindestens etwa 2,0 MPa·m^{0.5} und insbesondere mindestens etwa 2,3 MPa·m^{0.5} auf. Sie kann z.B. durch Wärmebehandlung eines entsprechenden Lithiumsilikatglases und insbesondere eines entsprechenden Lithiumsilikatglases mit Keimen gebildet werden.

In einer weiteren besonders bevorzugten Ausführungsform enthält die Glaskeramik Lithiumdisilikat als Hauptkristallphase. Insbesondere enthält die Glaskeramik mehr als 10 Vol.-%, bevorzugt mehr als 20 Vol.-% und besonders bevorzugt mehr als 30 Vol.-% an Lithiumdisilikat-Kristallen, bezogen auf die gesamte Glaskeramik. Diese Lithiumdisilikat-Glaskeramik zeichnet sich durch besonders gute mechanische Eigenschaften aus. Vorzugsweise weist sie eine Biegefestigkeit im Bereich von etwa 400 bis 700 MPa und/oder eine Bruchzähigkeit, gemessen als K_{IC} Wert, von mindestens etwa 2,0 MPa·m^{0.5} und insbesondere mindestens etwa 2,3 MPa ·m^{0,5} auf. Sie kann z.B. durch Wärmebehandlung der Lithiummetasilikat-Glaskeramik erzeugt werden. Sie kann aber auch durch Wärmebehandlung eines entsprechenden Lithiumsilikatglases oder eines entsprechenden Lithiumsilikatglases mit Keimen gebildet werden.

Als weitere Kristallphasen der Lithiumsilikat-Glaskeramik kommen insbesondere Li₃PO₄, SiO₂ und TiO₂ in Frage.

In einer weiteren Ausführungsform wird ein Lithiumsilikatglas verwendet. Dieses Lithiumsilikatglas enthält vorzugsweise Keime, die zur Ausbildung von Lithiummetasilikat- und/oder Lithiumdisilikatkristallen geeignet sind. Ein solches Lithiumsilikatglas mit Keimen kann insbesondere durch Wärmebehandlung eines entsprechenden Lithiumsilikatglases gebildet werden. Durch eine weitere Wärmebehandlung kann dann die erfindungsgemäße Lithiummetasilikat-Glaskeramik gebildet werden, die ihrerseits durch weitere Wärmebehandlung in die erfindungsgemäße Lithiumdisilikat-Glaskeramik umgewandelt werden kann, oder es kann auch bevorzugt direkt die erfindungsgemäße Lithiumdisilikat-Glaskeramik aus dem Glas mit Keimen gebildet werden. Mithin können das Lithiumsilikatglas, das Lithiumsilikatglas mit Keimen und die Lithiummetasilikat-Glaskeramik als Vorstufen zur Erzeugung einer erfindungsgemäßen hochfesten Lithiummeta- oder -disilikat-Glaskeramik angesehen werden. Vorzugsweise wird das Lithiumsilikatglas vor, während oder nach dem Aufbringen auf das Substrat in eine wie oben beschriebene Glaskeramik umgewandelt.

Zur Herstellung des Lithiumsilikatglases kann insbesondere so vorgegangen werden, dass eine Mischung von geeigneten Ausgangsmaterialien, wie z.B. Carbonaten, Oxiden, Phosphaten und Fluoriden, bei Temperaturen von insbesondere 1300 bis 1600°C für 2 bis 10 h erschmolzen wird. Zur Erzielung einer besonders hohen Homogenität wird die erhaltene Glasschmelze in Wasser gegossen, um ein Glasgranulat zu bilden, und das erhaltene Granulat wird dann erneut aufgeschmolzen. Die Schmelze kann dann in Formen gegossen werden, um Rohlinge des Lithiumsilikatglases, sogenannte Massivglasrohlinge oder monolithische Rohlinge, zu erzeugen. Es ist ebenfalls möglich, die Schmelze erneut in Wasser zu geben, um ein Granulat herzustellen. Dieses Granulat kann dann nach Mahlen und gegebenenfalls Zugabe weiterer Komponenten, wie Färbe- und Fluoreszenzmitteln, zu einem Rohling, einem sogenannten Pulverpressling, gepresst werden. Schließlich kann das Lithiumsilikatglas nach Granulierung auch zu einem Pulver verarbeitet werden.

Anschließend wird das Lithiumsilikatglas, z.B. in Form eines Massivglasrohlings, eines Pulverpresslings oder in Form eines Pulvers, mindestens einer Wärmebehandlung im Bereich von 450 bis 1050 °C unterzogen. Es ist bevorzugt, dass zunächst bei einer Temperatur im Bereich von 480 bis 580 °C, insbesondere 480 bis 560 °C und bevorzugt 480 bis 520 °C eine erste Wärmebehandlung durchgeführt wird, um ein Glas mit Keimen herzustellen, welche zur Bildung von Lithiummetasilikat- und/oder Lithiumdisilikatkristallen geeignet sind. Diese erste Wärmebehandlung wird bevorzugt für eine Dauer von 5 bis 120 Minuten, insbesondere 10 bis 60 Minuten und bevorzugt 10 bis 30 Minuten durchgeführt. Das Glas mit Keimen kann dann bevorzugt mindestens einer weiteren Temperaturbehandlung bei einer höheren Temperatur und insbesondere mehr als 580 °C unterworfen werden, um Kristallisation von Lithiummetasilikat oder von Lithiumdisilikat zu bewirken. Diese weitere Wärmebehandlung wird bevorzugt für eine Dauer von 10 bis 120 Minuten, insbesondere 10 bis 60 Minuten und besonders bevorzugt 20 bis 30 Minuten durchgeführt. Zur Kristallisation von Lithiummetasilikat erfolgt die weitere Wärmebehandlung üblicherweise bei 600 bis 950 °C, bevorzugt 620 bis 850 °C und ganz besonders bevorzugt 650 bis 750 °C. Zur Kristallisation von Lithiumdisilikat erfolgt die weitere Wärmebehandlung üblicherweise bei 750 bis 1050 °C, bevorzugt 800 bis 1000 °C, besonders bevorzugt 820 bis 950 °C und ganz besonders bevorzugt 850 bis 900 °C.

Die erfindungsgemäß verwendete Lithiumsilikat-Glaskeramik und das erfindungsgemäß verwendete Lithiumsilikatglas liegen insbesondere in Form von Pulvern, Granulaten oder Rohlingen, z.B. monolithische Rohlingen, wie Plättchen, Quadern oder Zylinder, oder Pulverpresslingen, in ungesinterter, teilgesinterter oder dichtgesinterter Form, vor. In diesen Formen können sie einfach weiterverarbeitet werden. Sie können aber auch in Form eines Überwurfs für dentale Restaurationen, wie insbesondere Kronen, vorliegen. Dabei ist es bevorzugt, dass die Glaskeramik oder das Glas durch maschinelle Bearbeitung oder Verpressen zur gewünschten Geometrie verformt werden.

Die erfindungsgemäß verwendete Lithiumsilikat-Glaskeramik und das erfindungsgemäß verwendete Lithiumsilikatglas eignen sich insbesondere zur Beschichtung von Oxidkeramiken, Metallen und Legierungen.

In einer bevorzugten Ausführungsform ist das Substrat eine Oxidkeramik. Dabei sind Zirkonoxidkeramiken besonders bevorzugt. Beispiele für geeignete Zirkonoxidkeramiken sind Keramiken auf Basis von polykristallinem tetragonalen Zirkoniumoxid (tetragonal zirconia polycrystal, TZP), bei denen die tetragonale Form durch Zusatz von Y₂O₃ und/oder CeO₂ stabilisiert ist.

Die Erfindung betrifft auch die Verwendung einer Lithiumsilikat-Glaskeramik oder eines Lithiumsilikatglases, die die folgenden Komponenten enthalten

| Komponente | Gew.-% |
|---|---|
| SiO₂ | 56,0 bis 73,0 |
| Li₂O | 13,0 bis 18,0 |
| Rb₂O | 3,0 bis 9,0 |
| Al₂O₃ | 2,0 bis 5,0 |
| P₂O₅ | 2,0 bis 6,0, |

zur Beschichtung eines Substrats, wobei das Substrat ein Metall oder eine Legierung ist. Dabei sind Nichtedelmetall-Legierungen und insbesondere Nichteisen-Legierungen, die für dentale Anwendungen geeignet sind, besonders bevorzugt. Beispiele für geeignete Legierungen sind insbesondere Legierungen vom Typ Ni-Cr, Co-Cr und Co-Cr-W.

Weiterhin ist es bevorzugt, dass das Substrat eine Dentalrestauration und insbesondere eine Brücke, ein Inlay, ein Onlay, ein Veneer, ein Abutment, eine Teilkrone, ein Krone oder ein Schale ist.

Die Erfindung betrifft auch ein Verfahren zur Beschichtung eines Substrats ausgewählt aus Oxidkeramiken, Metallen und Legierungen, bei dem eine wie oben beschriebene Lithiumsilikat-Glaskeramik oder ein wie oben beschriebenes Lithiumsilikatglas auf das Substrat aufgebracht werden. Vorzugsweise handelt es sich dabei um ein wie oben beschriebenes bevorzugtes Substrat.

In einer Ausführungsform des erfindungsgemäßen Verfahrens werden die Lithiumsilikat-Glaskeramik oder das Lithiumsilikatglas durch Aufsintern und bevorzugt durch Aufpressen auf das Substrat aufgebracht.

Beim Aufsintern werden die erfindungsgemäße Lithiumsilikat-Glaskeramik oder das erfindungsgemäße Lithiumsilikatglas in üblicher Weise, z.B. als Pulver, auf das zu beschichtende Material aufgebracht und anschließend bei erhöhter Temperatur gesintert.

Bei dem bevorzugten Aufpressen werden die erfindungsgemäße Lithiumsilikat-Glaskeramik oder das erfindungsgemäße Lithiumsilikatglas, z.B. in Form von Pulverpresslingen oder monolithischen Rohlingen, bei einer erhöhten Temperatur, von z.B. 700 bis 1200°C, in einen viskosen Zustand überführt und unter Anwendung von geringem Druck, z.B. 2 bis 10 bar, auf das Substrat aufgepresst. Hierzu können insbesondere die in der EP 231 773 A1 beschriebenen Verfahren und der dort offenbarte Pressofen eingesetzt werden. Ein geeigneter Ofen ist z.B. der Programat EP 5000 der Ivoclar Vivadent AG.

In einer anderen Ausführungsform des erfindungsgemäßen Verfahrens werden die Lithiumsilikat-Glaskeramik oder das Lithiumsilikatglas durch Fügen auf das Substrat aufgebracht. Geeignete Fügeverfahren sind an sich bekannt und umfassen beispielsweise Fügen mittels eines Glas- oder Glaskeramiklots, Fügen durch Kleben mittels eines Adhäsivs oder Dentalzements, Fügen durch Senken mittels einer Temperaturbehandlung, bei der die zu fügenden Werkstoffe erweicht werden, und Fügen durch Reibverschweißung oder Ansprengen.

In einer besonders bevorzugten Ausführungsform werden die Lithiumsilikat-Glaskeramik oder das Lithiumsilikatglas vor dem Fügen durch Heißpressen oder durch maschinelle Bearbeitung zu einer gewünschten Geometrie verformt.

Das Heißpressen erfolgt üblicherweise unter erhöhtem Druck und erhöhter Temperatur. Es ist bevorzugt, dass das Heißpressen bei einer Temperatur von 700 bis 1200°C erfolgt. Weiter ist es bevorzugt, das Heißpressen bei einem Druck von 2 bis 10 bar durchzuführen. Dabei wird durch viskoses Fließen des eingesetzten Materials die gewünschte Formänderung erreicht. Für das Heißpressen können die erfindungsgemäße Lithiummetasilikat-Glaskeramik, die erfindungsgemäße Lithiumdisilikat-Glaskeramik, das erfindungsgemäße Lithiumsilikatglas und insbesondere das erfindungsgemäße Lithiumsilikatglas mit Keimen verwendet werden. Dabei können die Glaskeramiken und Gläser insbesondere in Form von Rohlingen, z.B. Massivrohlingen oder Pulverpresslingen, z.B. in ungesinterter, teilgesinterter oder dichtgesinterter Form, eingesetzt werden.

Die maschinelle Bearbeitung erfolgt üblicherweise durch materialabtragende Verfahren und insbesondere durch Fräsen und/oder Schleifen. Es ist besonders bevorzugt, dass die maschinelle Bearbeitung im Rahmen eines CAD/CAM-Verfahrens durchgeführt wird. Für die maschinelle Bearbeitung können das Lithiumsilikatglas, das Lithiumsilikatglas mit Keimen, die Lithiummetasilikat- und die Lithiumdisilikat-Glaskeramik verwendet werden. Dabei können die Gläser und Glaskeramiken insbesondere in Form von Rohlingen, z.B. Massivrohlingen oder Pulverpresslingen, z.B. in ungesinterter, teilgesinterter oder dichtgesinterter Form, eingesetzt werden. Für die maschinelle Bearbeitung wird bevorzugt Lithiumsilikat-Glaskeramik insbesondere mit Lithiumdisilikat und bevorzugt mit Lithiummetasilikat als Hauptkristallphase verwendet. Die Lithiumsilikat-Glaskeramik kann auch in einer noch nicht vollständig kristallisierten Form eingesetzt werden, die durch Wärmebehandlung bei niedrigerer Temperatur erzeugt wurde. Dies bietet den Vorteil, dass eine leichtere maschinelle Bearbeitung und damit der Einsatz von einfacheren Apparaten zur maschinellen Bearbeitung möglich ist. Nach der maschinellen Bearbeitung eines solchen teilkristallisierten Materials wird dieses regelmäßig einer Wärmebehandlung bei höherer Temperatur und insbesondere 750 bis 1050 °C, bevorzugt 800 bis 950 °C und besonders bevorzugt etwa 850 bis 900 °C unterzogen, um weitere Kristallisation von Lithiummetasilikat oder vorzugsweise Lithiumdisilikat hervorzurufen.

Allgemein können die Lithiumsilikat-Glaskeramik oder das Lithiumsilikatglas nach der Verformung durch Heißpressen oder maschinelle Bearbeitung insbesondere noch wärmebehandelt werden, um eingesetzte Vorläufer, wie Lithiumsilikatglas, Lithiumsilikatglas mit Keimen oder Lithiummetasilikat-Glaskeramik, in Lithiummeta- und/oder - disilikat-Glaskeramik umzuwandeln, die Kristallisation von Lithiummeta- und/oder -disilikat zu steigern, oder die Porosität, z.B. eines eingesetzten porösen Pulverpresslings, zu vermindern. Es ist bevorzugt, dass nach Abschluss des Beschichtungsvorganges eine Beschichtung erhalten wird, die eine Lithiumsilikat-Glaskeramik mit Lithiummeta- und/oder -disilikat als Hauptkristallphase umfasst, da sie über besonders gute Eigenschaften verfügt. Dabei sind Glaskeramiken besonders bevorzugt, die die oben beschriebenen Kristallphasen und mechanischen Eigenschaften aufweisen.

Weiterhin betrifft die Erfindung einen Verbundwerkstoff, der eine wie oben definierte Lithiumsilikat-Glaskeramik oder ein wie oben definiertes Lithiumsilikatglas auf einem Substrat ausgewählt aus Oxidkeramiken, Metallen und Legierungen umfasst. Dabei sind alle Ausführungsformen bevorzugt, die auch für die erfindungsgemäß verwendete Lithiumsilikat-Glaskeramik, das erfindungsgemäß verwendete Lithiumsilikatglas sowie das Substrat als bevorzugt angegeben sind. Der Verbundwerkstoff kann insbesondere mittels des erfindungsgemäßen Verfahrens hergestellt werden.

Die Erfindung betrifft auch eine Lithiumsilikat-Glaskeramik, die die folgenden Komponenten enthält

| Komponente | Gew.-% |
|---|---|
| SiO₂ | 56,0 bis 72,5, insbesondere 56,9 bis 72,0 |
| Li₂O | 13,0 bis 19,0, insbesondere 14,2 bis 18,0 |
| Rb₂O | 3,0 bis 9,0, insbesondere 3,7 bis 7,7 |
| Al₂O₃ | 2,0 bis 5,0, insbesondere 2,5 bis 4,5 |
| P₂O₅ | 2,0 bis 6,0, insbesondere 3,1 bis 5,0 |
| ZrO₂ | 0 bis 4,5, insbesondere 0 bis 4,0 |
| Übergangsmetalloxid | 0 bis 7,5, insbesondere 0 bis 7,0, |

wobei das Übergangsmetalloxid ausgewählt ist aus der Gruppe bestehend aus Oxiden von Yttrium, Oxiden von Übergangsmetallen mit Ordnungszahl 41 bis 79 und Mischungen dieser Oxide.

Außerdem betrifft die Erfindung auch ein Lithiumsilikatglas, das die Komponenten der vorstehenden Glaskeramik enthält.

Das Lithiumsilikatglas und die Lithiumsilikat-Glaskeramik können darüber hinaus auch noch andere Komponenten enthalten, wie sie oben für die erfindungsgemäß verwendete Lithiumsilikat-Glaskeramik und das erfindungsgemäß verwendete Lithiumsilikatglas angegeben sind. Dabei sind alle Ausführungsformen bevorzugt, die auch für die erfindungsgemäß verwendete Lithiumsilikat-Glaskeramik und das erfindungsgemäß verwendete Lithiumsilikatglas als bevorzugt angegeben sind.

Die Erfindung wird im Folgenden anhand von Beispielen näher erläutert.

### Beispiele

Es wurden insgesamt 15 erfindungsgemäße Gläser und Glaskeramiken mit den in Tabelle I angegebenen Zusammensetzungen über Erschmelzung entsprechender Ausgangsgläser und anschließende Wärmebehandlung zur gesteuerten Keimbildung und Kristallisation hergestellt.

Dazu wurden zunächst die Ausgangsgläser im 100 bis 200 g Maßstab aus üblichen Rohstoffen bei 1450 bis 1550 °C erschmolzen, wobei das Erschmelzen sehr gut ohne Bildung von Blasen oder Schlieren möglich war. Durch Eingießen der Ausgangsgläser in Wasser wurden Glasfritten hergestellt, die zur Homogenisierung anschließend ein zweites Mal bei 1450 bis 1550 °C für 1 bis 3 h geschmolzen wurden. Die erhaltenen Glasschmelzen wurden dann in vorgewärmte Formen gegossen, um Glasmonolithe zu erzeugen. Alle Glasmonolithe erwiesen sich als transparent.

Die Glasmonolithe wurden dann durch thermische Behandlung zu erfindungsgemäßen Gläsern und Glaskeramiken umgewandelt. Die angewendeten thermischen Behandlungen zur gesteuerten Keimbildung und gesteuerten Kristallisation sind ebenfalls in Tabelle I angegeben. Dabei bedeuten
- T_{N} und t_{N}: Angewendete Temperatur und Zeit für Keimbildung
- T_{C} und t_{C}: Angewendete Temperatur und Zeit für erste Kristallisation
- T_{FC} und t_{FC}: Angewendete Temperatur und Zeit für endgültige Kristallisation
- Tₚᵣₑₛₛ und tₚᵣₑₛₛ: Angewendete Temperatur und Zeit für Heißpressen

Es ist ersichtlich, dass eine erste Wärmebehandlung im Bereich von 470 bis 500 °C zur Bildung von Lithiumsilikat-Gläsern mit Keimen führte und diese Gläser durch weitere Wärmebehandlung bei 600 bis 710 °C (Beispiele 1-6, 8-9 und 13) zu Glaskeramiken mit Lithiummetasilikat als Hauptkristallphase bzw. durch Wärmebehandlung bei 880 °C (Beispiele 7 und 14) direkt zu Glaskeramiken mit Lithiumdisilikat als Hauptkristallphase kristallisierten, wie durch Röntgenbeugungsuntersuchungen festgestellt wurde. Eine abschließende Wärmebehandlung bei einer Temperatur von 860 bis 950 °C (Beispiele 1, 3-4, 6-8, 10-12 und 14) führte schließlich zur Bildung von Glaskeramiken mit Lithiumdisilikat als Hauptkristallphase. Eine abschließende Wärmebehandlung bei einer Temperatur von lediglich 820 bis 840 °C (Beispiele 2, 5, 9 und 13) führte dagegen zur Bildung von Glaskeramiken mit Lithiummetasilikat als Hauptkristallphase.

Die erzeugten Lithiumdisilikat-Glaskeramiken hatten hohe Bruchzähigkeiten, gemessen als kritischer Spannungsintensitätsfaktor K_{IC} nach der SEVNB-Methode, von mehr als 2 MPa•m^{0,5} und insbesondere sogar mindestens 2,3 MPa•m^{0,5}.

Auch die Biaxialfestigkeit σ_{B} war mit mehr als 400 MPa und bis zu mehr als 600 MPa hoch. Sie wurde gemäß Dentalnorm ISO 6872 (2008) an Prüfkörpern bestimmt, die durch maschinelle Bearbeitung der jeweiligen Lithiumdisilikat-Glaskeramik hergestellt wurden. Zur Bearbeitung wurde eine CEREC-InLab Maschine (Sirona, Bensheim) verwendet.

Ebenfalls konnten sie durch Heißpressen als Beschichtungen insbesondere auf Oxidkeramikrestaurationen oder Metallrestaurationen aufgebracht werden, z.B. um diese in gewünschter Weise zu verblenden.

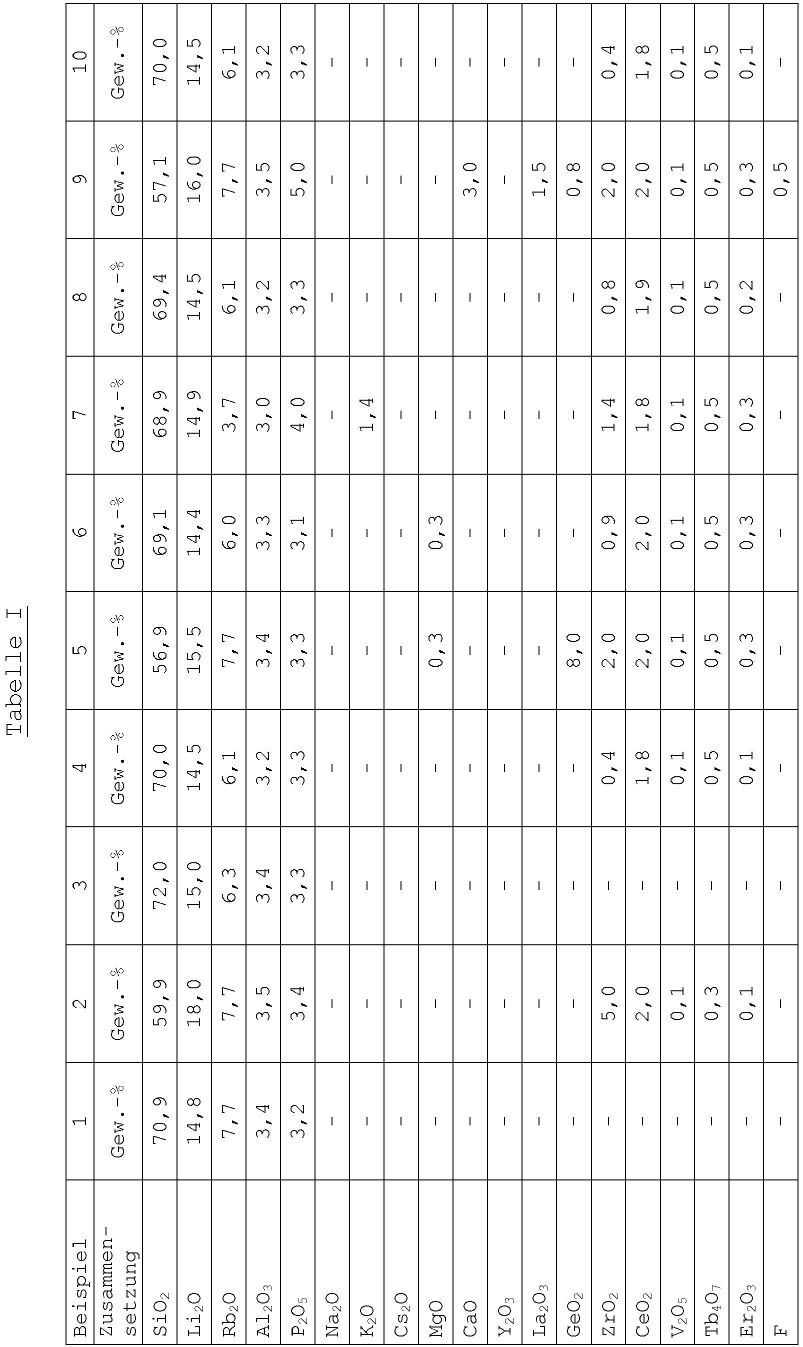

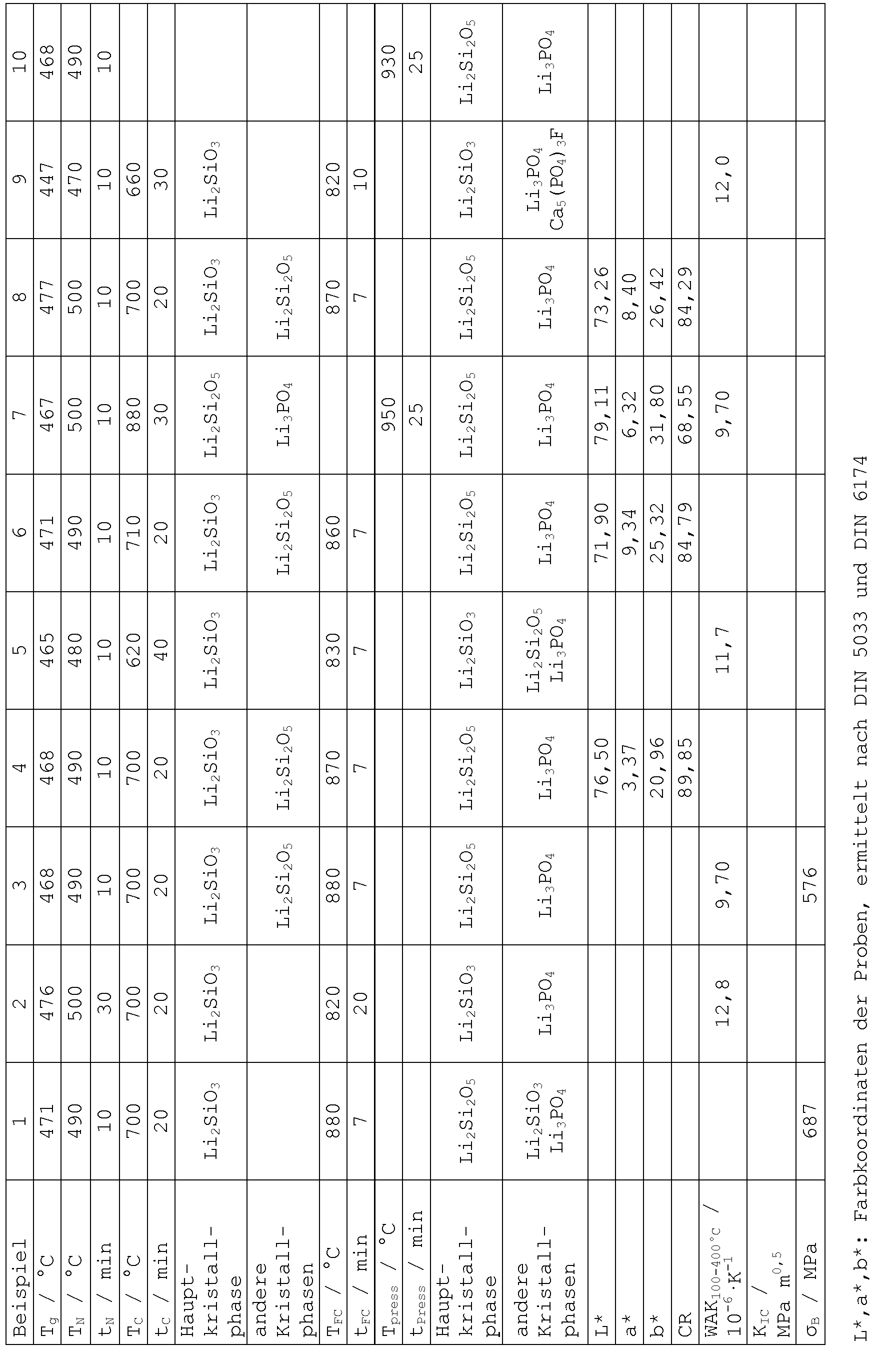

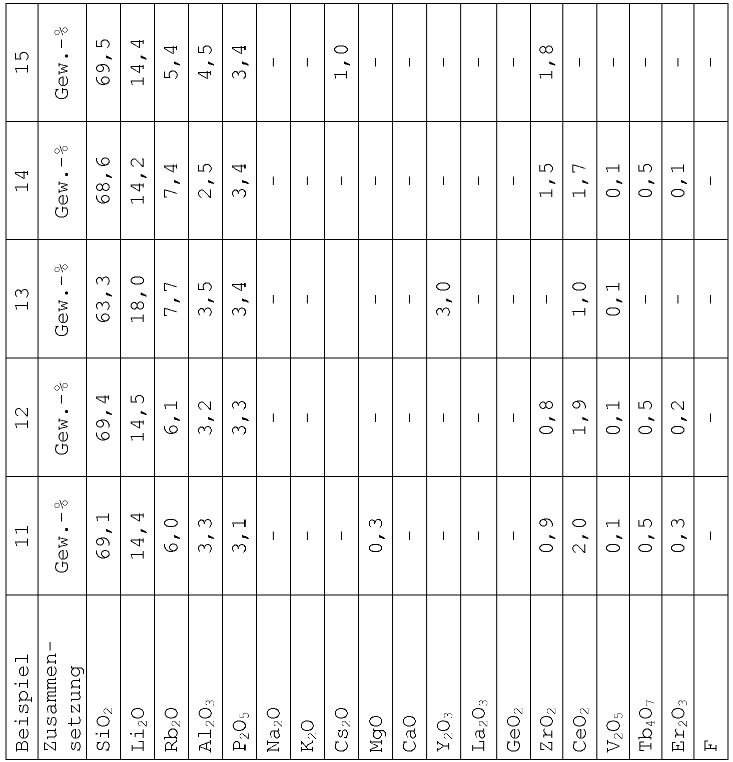

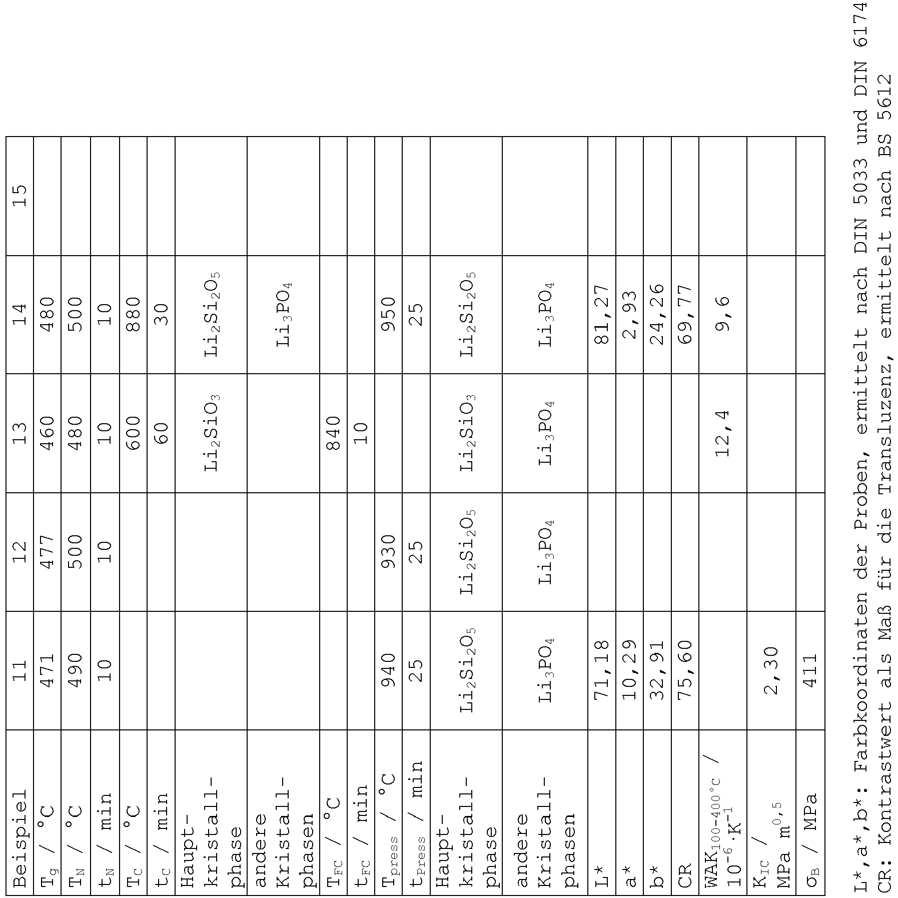

## Patentansprüche

1. Verwendung einer Lithiumsilikat-Glaskeramik oder eines Lithiumsilikatglases, die die folgenden Komponenten enthalten
| Komponente | Gew.-% |
|---|---|
| SiO₂ | 56,0 bis 72,0 |
| Li₂O | 13,0 bis 18,0 |
| Rb₂O | 3,0 bis 9,0 |
| Al₂O₃ | 2,0 bis 5,0 |
| P₂O₅ | 2,0 bis 6,0 |
| ZrO₂ | 0 bis 4,5, |
zur Beschichtung eines Substrats ausgewählt aus Oxidkeramiken, Metallen und Legierungen.

2. Verwendung nach Anspruch 1, bei der die Lithiumsilikat-Glaskeramik oder das Lithiumsilikatglas mindestens eine und bevorzugt alle der folgenden Komponenten in den angegebenen Mengen enthalten
| Komponente | Gew.-% |
|---|---|
| SiO₂ | 56,9 bis 72,0 |
| Li₂O | 14,2 bis 18,0 |
| Rb₂O | 3,7 bis 7,7 |
| Al₂O₃ | 2,5 bis 4,5 |
| P₂O₅ | 3,1 bis 5,0 |
| ZrO₂ | 0 bis 4,0 |
| Übergangsmetalloxid | 0 bis 7,5, insbesondere 0 bis 7,0, |
wobei das Übergangsmetalloxid ausgewählt ist aus der Gruppe bestehend aus Oxiden von Yttrium, Oxiden von Übergangsmetallen mit Ordnungszahl 41 bis 79 und Mischungen dieser Oxide.

3. Verwendung nach Anspruch 1 oder 2, bei der die Lithiumsilikat-Glaskeramik oder das Lithiumsilikatglas 58,0 bis 72,0, insbesondere 60,0 bis 71,0 und bevorzugt 63,0 bis 70,0 Gew.-% SiO₂ enthalten.

4. Verwendung nach einem der Ansprüche 1 bis 3, bei der die Lithiumsilikat-Glaskeramik oder das Lithiumsilikatglas 3,7 bis 7,7 Gew.-%, insbesondere 5,1 bis 7,7 Gew.-% und bevorzugt 6,1 bis 7,4 Gew.-% Rb₂O enthalten.

5. Verwendung nach einem der Ansprüche 1 bis 4, bei der die Lithiumsilikat-Glaskeramik oder das Lithiumsilikatglas 2,5 bis 4,0 Gew.-%, insbesondere 3,0 bis 3,5 Gew.-% und bevorzugt 3,2 bis 3,4 Gew.-% Al₂O₃ enthalten.

6. Verwendung nach einem der Ansprüche 1 bis 5, bei der die Lithiumsilikat-Glaskeramik oder das Lithiumsilikatglas weniger als 2,5 Gew.-%, insbesondere weniger als 1,5 Gew.-%, bevorzugt weniger als 1,0 Gew.-%, besonders bevorzugt weniger als 0,5 Gew.-% Cs₂O enthalten und am meisten bevorzugt im Wesentlichen frei von Cs₂O sind.

7. Verwendung nach einem der Ansprüche 1 bis 6, bei der die Lithiumsilikat-Glaskeramik oder das Lithiumsilikatglas weniger als 4,0 Gew.-%, insbesondere weniger als 3,5 Gew.-%, bevorzugt weniger als 3,0 Gew.-%, besonders bevorzugt weniger als 2,5 Gew.-% und am meisten bevorzugt weniger als 2,0 Gew.-% Na₂O und/oder K₂O enthalten.

8. Verwendung nach einem der Ansprüche 1 bis 7, bei der die Lithiumsilikat-Glaskeramik oder das Lithiumsilikatglas weniger als 3,8 Gew.-%, insbesondere weniger als 2,5 Gew.-%, bevorzugt weniger als 1,5 Gew.-%, besonders bevorzugt weniger als 0,5 Gew.-% BaO enthalten und am meisten bevorzugt im Wesentlichen frei von BaO sind.

9. Verwendung nach einem der Ansprüche 1 bis 8, bei der eine Lithiumsilikat-Glaskeramik verwendet wird, die Lithiummetasilikat als Hauptkristallphase enthält und vorzugsweise eine Biegefestigkeit im Bereich von etwa 180 bis 300 MPa und/oder eine Bruchzähigkeit, gemessen als K_{IC} Wert, von mindestens etwa 2,0 MPa·m^{0.5} und insbesondere mindestens etwa 2,3 MPa·m^{0.5} aufweist.

10. Verwendung nach einem der Ansprüche 1 bis 9, bei der eine Lithiumsilikat-Glaskeramik verwendet wird, die Lithiumdisilikat als Hauptkristallphase enthält und vorzugsweise eine Biegefestigkeit im Bereich von etwa 400 bis 700 MPa und/oder eine Bruchzähigkeit, gemessen als K_{IC} Wert, von mindestens etwa 2,0 MPa·m^{0.5} und insbesondere mindestens etwa 2,3 MPa·m^{0.5} aufweist.

11. Verwendung nach einem der Ansprüche 1 bis 8, bei der ein Lithiumsilikatglas verwendet wird, wobei das Lithiumsilikatglas vorzugsweise Keime enthält, die zur Ausbildung von Lithiummetasilikat- und/oder Lithiumdisilikatkristallen geeignet sind.

12. Verwendung nach einem der Ansprüche 1 bis 11, bei der das Substrat eine Oxidkeramik und insbesondere eine Zirkonoxidkeramik ist.

13. Verwendung einer Lithiumsilikat-Glaskeramik oder eines Lithiumsilikatglases, die die folgenden Komponenten enthalten
| Komponente | Gew.-% |
|---|---|
| SiO₂ | 56,0 bis 73,0 |
| Li₂O | 13,0 bis 18,0 |
| Rb₂O | 3,0 bis 9,0 |
| Al₂O₃ | 2,0 bis 5,0 |
| P₂O₅ | 2,0 bis 6,0, |
zur Beschichtung eines Substrats, wobei das Substrat ein Metall oder eine Legierung und insbesondere eine Nichtedelmetall-Legierung ist.

14. Verwendung nach einem der Ansprüche 1 bis 13, bei der das Substrat eine Dentalrestauration und insbesondere eine Brücke, ein Inlay, ein Onlay, ein Veneer, ein Abutment, eine Teilkrone, ein Krone oder ein Schale ist.

15. Verfahren zur Beschichtung eines Substrats ausgewählt aus Oxidkeramiken, Metallen und Legierungen und insbesondere eines Substrats, das wie in einem der Ansprüche 12 bis 14 definiert ist, bei dem eine Lithiumsilikat-Glaskeramik oder ein Lithiumsilikatglas auf das Substrat aufgebracht werden, die wie in einem der Ansprüche 1 bis 11 definiert sind.

16. Verfahren nach Anspruch 15, bei dem die Lithiumsilikat-Glaskeramik oder das Lithiumsilikatglas durch Aufsintern und bevorzugt durch Aufpressen auf das Substrat aufgebracht werden.

17. Verfahren nach Anspruch 15, bei dem die Lithiumsilikat-Glaskeramik oder das Lithiumsilikatglas durch Fügen auf das Substrat aufgebracht werden.

18. Verfahren nach Anspruch 17, bei dem die Lithiumsilikat-Glaskeramik oder das Lithiumsilikatglas vor dem Fügen durch maschinelle Bearbeitung oder durch Heißpressen zu einer gewünschten Geometrie verformt werden.

19. Verfahren nach einem der Ansprüche 15 bis 18, bei dem eine Beschichtung erhalten wird, die eine Lithiumsilikat-Glaskeramik umfasst, welche Lithiummetasilikat als Hauptkristallphase enthält, und vorzugsweise eine Biegefestigkeit im Bereich von etwa 180 bis 300 MPa und/oder eine Bruchzähigkeit, gemessen als K_{IC} Wert, von mindestens etwa 2,0 MPa ·m^{0.5} und insbesondere mindestens etwa 2,3 MPa ·m^{0.5} aufweist.

20. Verfahren nach einem der Ansprüche 15 bis 19, bei dem eine Beschichtung erhalten wird, die eine Lithiumsilikat-Glaskeramik umfasst, welche Lithiumdisilikat als Hauptkristallphase enthält, und vorzugsweise eine Biegefestigkeit im Bereich von etwa 400 bis 700 MPa und/oder eine Bruchzähigkeit, gemessen als K_{IC} Wert, von mindestens etwa 2,0 MPa·m^{0.5} und insbesondere mindestens etwa 2,3 MPa·m^{0.5} aufweist.

21. Verbundwerkstoff, der eine Lithiumsilikat-Glaskeramik oder ein Lithiumsilikatglas, die wie in einem der Ansprüche 1 bis 10 definiert sind, auf einem Substrat ausgewählt aus Oxidkeramiken, Metallen und Legierungen und insbesondere auf einem Substrat umfasst, das wie in einem der Ansprüche 12 bis 14 definiert ist.

22. Lithiumsilikat-Glaskeramik, die wie in einem der Ansprüche 1 bis 10 definiert ist und die die folgenden Komponenten in den angegebenen Mengen enthält
| Komponente | Gew.-% |
|---|---|
| SiO₂ | 56,0 bis 72,5, insbesondere 56,9 bis 72,0 |
| Li₂O | 13,0 bis 18,0, insbesondere 14,2 bis 18,0 |
| Rb₂O | 3,0 bis 9,0, insbesondere 3,7 bis 7,7 |
| Al₂O₃ | 2,0 bis 5,0, insbesondere 2,5 bis 4,5 |
| P₂O₅ | 2,0 bis 6,0, insbesondere 3,1 bis 5,0 |
| ZrO₂ | 0 bis 4,5, insbesondere 0 bis 4,0 |
| Übergangsmetalloxid | 0 bis 7,5, insbesondere 0 bis 7,0, |
wobei das Übergangsmetalloxid ausgewählt ist aus der Gruppe bestehend aus Oxiden von Yttrium, Oxiden von Übergangsmetallen mit Ordnungszahl 41 bis 79 und Mischungen dieser Oxide.

23. Lithiumsilikatglas, das die Komponenten der Glaskeramik nach Anspruch 22 enthält.

## Claims

1. Use of a lithium silicate glass ceramic or a lithium silicate glass which comprise the following components
| Component | wt.-% |
|---|---|
| SiO₂ | 56.0 to 72.0 |
| Li₂O | 13.0 to 18.0 |
| Rb₂O | 3.0 to 9.0 |
| Al₂O₃ | 2.0 to 5.0 |
| P₂O₅ | 2.0 to 6.0 |
| ZrO₂ | 0 to 4.5, |
for coating a substrate selected from oxide ceramics, metals and alloys.

2. Use according to claim 1, in which the lithium silicate glass ceramic or the lithium silicate glass comprise at least one and preferably all of the following components in the given amounts
| Component | wt.-% |
|---|---|
| SiO₂ | 56.9 to 72.0 |
| Li₂O | 14.2 to 18.0 |
| Rb₂O | 3.7 to 7.7 |
| Al₂O₃ | 2.5 to 4.5 |
| P₂O₅ | 3.1 to 5.0 |
| ZrO₂ | 0 to 4.0 |
| Transition metal oxide | 0 to 7.5, in particular 0 to 7.0, |
wherein the transition metal oxide is selected from the group consisting of oxides of yttrium, oxides of transition metals with an atomic number from 41 to 79 and mixtures of these oxides.

3. Use according to claim 1 or 2, in which the lithium silicate glass ceramic or the lithium silicate glass comprise 58.0 to 72.0, in particular 60.0 to 71.0 and preferably 63.0 to 70.0 wt.-% SiO₂.

4. Use according to any one of claims 1 to 3, in which the lithium silicate glass ceramic or the lithium silicate glass comprise 3.7 to 7.7 wt.-%, in particular 5.1 to 7.7 wt.-% and preferably 6.1 to 7.4 wt.-% Rb₂O.

5. Use according to any one of claims 1 to 4, in which the lithium silicate glass ceramic or the lithium silicate glass comprise 2.5 to 4.0 wt.-%, in particular 3.0 to 3.5 wt.-% and preferably 3.2 to 3.4 wt.-% Al₂O₃.

6. Use according to any one of claims 1 to 5, in which the lithium silicate glass ceramic or the lithium silicate glass comprise less than 2.5 wt.-%, in particular less than 1.5 wt.-%, preferably less than 1.0 wt.-%, particularly preferably less than 0.5 wt.-% Cs₂O and is most preferably substantially free of Cs₂O.

7. Use according to any one of claims 1 to 6, in which the lithium silicate glass ceramic or the lithium silicate glass comprise less than 4.0 wt.-%, in particular less than 3.5 wt.-%, preferably less than 3.0 wt.-%, particularly preferably less than 2.5 wt.-% and most preferably less than 2.0 wt.-% Na₂O and/or K₂O.

8. Use according to any one of claims 1 to 7, in which the lithium silicate glass ceramic or the lithium silicate glass comprise less than 3.8 wt.-%, in particular less than 2.5 wt.-%, preferably less than 1.5 wt.-%, particularly preferably less than 0.5 wt.-% BaO and is most preferably substantially free of BaO.

9. Use according to any one of claims 1 to 8, in which a lithium silicate glass ceramic is used which comprises lithium metasilicate as main crystal phase and preferably has a bending strength in the range of about 180 to 300 MPa and/or a fracture toughness, measured as K_{IC} value, of at least about 2.0 MPa·m^{0.5} and in particular at least about 2.3 MPa·m^{0.5}.

10. Use according to one of claims 1 to 9, in which a lithium silicate glass ceramic is used which comprises lithium disilicate as main crystal phase and preferably has a bending strength in the range of about 400 to 700 MPa and/or a fracture toughness, measured as K_{IC} value, of at least about 2.0 MPa·m^{0.5} and in particular at least about 2.3 MPa·m^{0.5}.

11. Use according to any one of claims 1 to 8, in which a lithium silicate glass is used, wherein the lithium silicate glass preferably comprises nuclei which are suitable for forming lithium metasilicate and/or lithium disilicate crystals.

12. Use according to any one of claims 1 to 11, in which the substrate is an oxide ceramic and in particular a zirconium oxide ceramic.

13. Use of a lithium silicate glass ceramic or a lithium silicate glass which comprise the following components
| Component | wt.-% |
|---|---|
| SiO₂ | 56.0 to 73.0 |
| Li₂O | 13.0 to 18.0 |
| Rb₂O | 3.0 to 9.0 |
| Al₂O₃ | 2.0 to 5.0 |
| P₂O₅ | 2.0 to 6.0 |
for coating a substrate, in which the substrate is a metal or an alloy and in particular a non-precious metal alloy.

14. Use according to any one of claims 1 to 13, in which the substrate is a dental restoration and in particular a bridge, an inlay, an onlay, a veneer, an abutment, a partial crown, a crown or a facet.

15. Process for coating a substrate selected from oxide ceramics, metals and alloys, and in particular a substrate which is as defined in any one of claims 12 to 14, in which a lithium silicate glass ceramic or a lithium silicate glass, as defined in any one of claims 1 to 11, is applied to the substrate.

16. Process according to claim 15, in which the lithium silicate glass ceramic or the lithium silicate glass is applied to the substrate by sintering and preferably by pressing-on.

17. Process according to claim 15, in which the lithium silicate glass ceramic or the lithium silicate glass is applied to the substrate by joining.

18. Process according to claim 17, in which the lithium silicate glass ceramic or the lithium silicate glass is shaped to a desired geometry by machining or by hot pressing before joining.

19. Process according to any one of claims 15 to 18, in which a coating is obtained which comprises a lithium silicate glass ceramic that comprises lithium metasilicate as main crystal phase, and which preferably has a bending strength in the range of about 180 to 300 MPa and/or a fracture toughness, measured as K_{IC} value, of at least about 2.0 MPa·m^{0.5} and in particular at least about 2.3 MPa·m^{0.5}.

20. Process according to any one of claims 15 to 19, in which a coating is obtained which comprises a lithium silicate glass ceramic that comprises lithium disilicate as main crystal phase, and which preferably has a bending strength in the range of about 400 to 700 MPa and/or a fracture toughness, measured as K_{IC} value, of at least about 2.0 MPa·m^{0.5} and in particular at least about 2.3 MPa·m^{0.5}.

21. Composite material which comprises a lithium silicate glass ceramic or a lithium silicate glass, as defined in any one of claims 1 to 10, on a substrate selected from oxide ceramics, metals and alloys, and in particular on a substrate which is as defined in any one of claims 12 to 14.

22. Lithium silicate glass ceramic, which is as defined in any one of claims 1 to 10 and which comprises the following components in the given amounts
| Component | wt.-% |
|---|---|
| SiO₂ | 56.0 to 72.5, in particular 56.9 to 72.0 |
| Li₂O | 13.0 to 19.0, in particular 14.2 to 18.0 |
| Rb₂O | 3.0 to 9.0, in particular 3.7 to 7.7 |
| Al₂O₃ | 2.0 to 5.0, in particular 2.5 to 4.5 |
| P₂O₅ | 2.0 to 6.0, in particular 3.1 to 5.0 |
| ZrO₂ | 0 to 4.5, in particular 0 to 4.0 |
| Transition metal oxide | 0 to 7.5, in particular 0 to 7.0, |
wherein the transition metal oxide is selected from the group consisting of oxides of yttrium, oxides of transition metals with an atomic number from 41 to 79 and mixtures of these oxides.

23. Lithium silicate glass, which comprises the components of the glass ceramic according to claim 22.

## Revendications

1. Utilisation d'une vitrocéramique à base de silicate de lithium ou d'un verre à base de silicate de lithium, qui contiennent les composants suivants
| Composant | % en poids |
|---|---|
| SiO₂ | 56,0 à 72,0 |
| Li₂O | 13,0 à 18,0 |
| Rb₂O | 3,0 à 9,0 |
| Al₂O₃ | 2,0 à 5,0 |
| P₂O₅ | 2,0 à 6,0 |
| ZrO₂ | 0 à 4,5, |
pour le revêtement d'un support choisi parmi les céramiques à base d'oxydes, les métaux et alliages.

2. Utilisation selon la revendication 1, dans laquelle la vitrocéramique à base de silicate de lithium ou le verre à base de silicate de lithium contiennent au moins l'un et de préférence la totalité des composants suivants en les quantités indiquées
| Composant | % en poids |
|---|---|
| SiO₂ | 56,9 à 72,0 |
| Li₂O | 14,2 à 18,0 |
| Rb₂O | 3,7 à 7,7 |
| Al₂O₃ | 2,5 à 4,5 |
| P₂O₅ | 3,1 à 5,0 |
| ZrO₂ | 0 à 4,0 |
| oxyde de métal de transition | 0 à 7,5, en particulier 0 à 7,0, |
l'oxyde de métal de transition étant choisi dans l'ensemble constitué par les oxydes d'yttrium, les oxydes de métaux de transition de nombre atomique 41 à 79 et les mélanges de ces oxydes.

3. Utilisation selon la revendication 1 ou 2, dans laquelle la vitrocéramique à base de silicate de lithium ou le verre à base de silicate de lithium contiennent 58,0 à 72,0, en particulier 60,0 à 71,0 et de préférence 63,0 à 70,0 % en poids de SiO₂.

4. Utilisation selon l'une quelconque des revendications 1 à 3, dans laquelle la vitrocéramique à base de silicate de lithium ou le verre à base de silicate de lithium contiennent 3,7 à 7,7 % en poids, en particulier 5,1 à 7,7 % en poids et de préférence 6,1 à 7,4 % en poids de Rb₂O.

5. Utilisation selon l'une quelconque des revendications 1 à 4, dans laquelle la vitrocéramique à base de silicate de lithium ou le verre à base de silicate de lithium contiennent 2,5 à 4,0 % en poids, en particulier 3,0 à 3,5 % en poids et de préférence 3,2 à 3,4 % en poids d'Al₂O₃.

6. Utilisation selon l'une quelconque des revendications 1 à 5, dans laquelle la vitrocéramique à base de silicate de lithium ou le verre à base de silicate de lithium contiennent moins de 2,5 % en poids, en particulier moins de 1,5 % en poids, de préférence moins de 1,0 % en poids, de façon particulièrement préférée moins de 0,5 % en poids de Cs₂O et de façon tout particulièrement préférée sont pratiquement exempts de Cs₂O.

7. Utilisation selon l'une quelconque des revendications 1 à 6, dans laquelle la vitrocéramique à base de silicate de lithium ou le verre à base de silicate de lithium contiennent moins de 4,0 % en poids, en particulier moins de 3,5 % en poids, de préférence moins de 3,0 % en poids, de façon particulièrement préférée moins de 2,5 % en poids et de façon tout particulièrement préférée moins de 2,0 % en poids de Na₂O et/ou K₂O.

8. Utilisation selon l'une quelconque des revendications 1 à 7, dans laquelle la vitrocéramique à base de silicate de lithium ou le verre à base de silicate de lithium contiennent moins de 3,8 % en poids, en particulier moins de 2,5 % en poids, de préférence moins de 1,5 % en poids, de façon particulièrement préférée moins de 0,5 % en poids de BaO et de façon tout particulièrement préférée sont pratiquement exempts de BaO.

9. Utilisation selon l'une quelconque des revendications 1 à 8, dans laquelle on utilise une vitrocéramique à base de silicate de lithium qui contient du métasilicate de lithium en tant que phase cristalline principale et présente de préférence une résistance à la flexion dans la plage d'environ 180 à 300 MPa et/ou une résistance à la rupture, mesurée en tant que valeur K_{IC}, d'au moins environ 2,0 MPa.m^{0,5} et en particulier d'au moins environ 2,3 MPa.m^{0,5}.

10. Utilisation selon l'une quelconque des revendications 1 à 9, dans laquelle on utilise une vitrocéramique à base de silicate de lithium qui contient du disilicate de lithium en tant que phase cristalline principale et présente de préférence une résistance à la flexion dans la plage d'environ 400 à 700 MPa et/ou une résistance à la rupture, mesurée en tant que valeur K_{IC}, d'au moins environ 2,0 MPa.m^{0,5} et en particulier d'au moins environ 2,3 MPa.m^{0,5}.

11. Utilisation selon l'une quelconque des revendications 1 à 8, dans laquelle on utilise un verre à base de silicate de lithium, le verre à base de silicate de lithium contenant de préférence des germes qui sont appropriés à la formation de cristaux de métasilicate de lithium et/ou de cristaux de disilicate de lithium.

12. Utilisation selon l'une quelconque des revendications 1 à 11, dans laquelle le support est une céramique à base d'oxyde et en particulier une céramique à base d'oxyde de zirconium.

13. Utilisation d'une vitrocéramique à base de silicate de lithium ou d'un verre à base de silicate de lithium, qui contiennent les composants suivants
| Composant | % en poids |
|---|---|
| SiO₂ | 56,0 à 73,0 |
| Li₂O | 13,0 à 18,0 |
| Rb₂O | 3,0 à 9,0 |
| Al₂O₃ | 2,0 à 5,0 |
| P₂O₅ | 2,0 à 6,0, |
pour le revêtement d'un support, le support étant un métal ou un alliage et en particulier un alliage de métaux non précieux.

14. Utilisation selon l'une quelconque des revendications 1 à 13, dans laquelle le support est une restauration dentaire et en particulier un bridge, un inlay, un onlay, un placage, un pilier, une couronne partielle, une couronne ou une facette.

15. Procédé pour le revêtement d'un support choisi parmi les céramiques à base d'oxydes, les métaux et alliages et en particulier d'un support qui est tel que défini dans l'une quelconque des revendications 12 à 14, dans lequel on applique sur le support une vitrocéramique à base de silicate de lithium ou un verre à base de silicate de lithium, qui sont tels que définis dans l'une quelconque des revendications 1 à 11.

16. Procédé selon la revendication 15, dans lequel on applique sur le support la vitrocéramique à base de silicate de lithium ou le verre à base de silicate de lithium par application par frittage et de préférence par application par pressage.

17. Procédé selon la revendication 15, dans lequel on applique sur le support la vitrocéramique à base de silicate de lithium ou le verre à base de silicate de lithium par assemblage.

18. Procédé selon la revendication 17, dans lequel avant l'assemblage on met en forme à une géométrie désirée la vitrocéramique à base de silicate de lithium ou le verre à base de silicate de lithium par usinage mécanique ou par pressage à chaud.

19. Procédé selon l'une quelconque des revendications 15 à 18, dans lequel on obtient un revêtement qui comprend une vitrocéramique à base de silicate de lithium, qui contient du métasilicate de lithium en tant que phase cristalline principale et présente de préférence une résistance à la flexion dans la plage d'environ 180 à 300 MPa et/ou une résistance à la rupture, mesurée en tant que valeur K_{IC}, d'au moins environ 2,0 MPa.m^{0,5} et en particulier d'au moins environ 2,3 MPa.m^{0,5}.

20. Procédé selon l'une quelconque des revendications 15 à 19, dans lequel on obtient un revêtement qui comprend une vitrocéramique à base de silicate de lithium, qui contient du disilicate de lithium en tant que phase cristalline principale et présente de préférence une résistance à la flexion dans la plage d'environ 400 à 700 MPa et/ou une résistance à la rupture, mesurée en tant que valeur K_{IC}, d'au moins environ 2,0 MPa.m^{0,5} et en particulier d'au moins environ 2,3 MPa.m^{0,5}.

21. Matériau composite, qui comprend une vitrocéramique à base de silicate de lithium ou un verre à base de silicate de lithium qui sont tels que définis dans l'une quelconque des revendications 1 à 10, sur un support choisi parmi les céramiques à base d'oxydes, les métaux et alliages et en particulier sur un support qui est tel que défini dans l'une quelconque des revendications 12 à 14.

22. Vitrocéramique à base de silicate de lithium, qui est telle que définie dans l'une quelconque des revendications 1 à 10 et qui contient les composants suivants en les quantités indiquées
| Composant | % en poids |
|---|---|
| SiO₂ | 56,0 à 72,5, en particulier 56,9 à 72,0 |
| Li₂O | 13,0 à 18,0, en particulier 14,2 à 18,0 |
| Rb₂O | 3,0 à 9,0, en particulier 3,7 à 7,7 |
| Al₂O₃ | 2,0 à 5,0, en particulier 2,5 à 4,5 |
| P₂O₅ | 2,0 à 6,0, en particulier 3,1 à 5,0 |
| ZrO₂ | 0 à 4,5, en particulier 0 à 4,0 |
| oxyde de métal de transition | 0 à 7,5, en particulier 0 à 7,0 |
l'oxyde de métal de transition étant choisi dans l'ensemble constitué par les oxydes d'yttrium, les oxydes de métaux de transition de nombre atomique 41 à 79 et les mélanges de ces oxydes.

23. Verre à base de silicate de lithium, qui contient les composants de la vitrocéramique selon la revendication 22.
